# EUROPÄISCHE PATENTSCHRIFT

(11) **EP 2 354 233 B1**
(45) Veröffentlichungstag und Bekanntmachung des Hinweises auf die Patenterteilung: **19.11.2014**
(21) Anmeldenummer: 11002905.5
(22) Anmeldetag: 16.06.2007
(51) Int. Cl.: C12N 15/82, C07K 14/415, A01H 5/00, A01H 5/10

(54) **Pathogen induzierbarer synthetischer Promotor**
Pathogen inducible synthetic promoter
Promoteur synthétique pouvant être induit par un pathogène

(30) Priorität: 22.06.2006 DE 102006029129
(43) Veröffentlichungstag der Anmeldung: 10.08.2011
(62) Teilanmeldung aus: 07764382.3
(73) Patentinhaber: KWS Saat AG, 37555 Einbeck (DE)
(72) Erfinder: Schmidt, Klaus Dr., 31246 Lahnstedt/Gr. Lafferde (DE)
(74) Vertreter: Westhoff, Markus

(56) Entgegenhaltungen:
- WO-A-00/29592
- WO-A-98/03536
- WO-A-03/083042
- WO-A-2005/123919
- WO-A1-01/07635
- WO-A2-02/22675
- WO-A2-02/34927
- US-A1- 2004 083 501
- DATABASE EMBL [Online] 8. Mai 2005 (2005-05-08), "Zea mays PR4 gene promoter", XP002474396, gefunden im EBI accession no. EMBL:AJ969166 Database accession no. AJ969166 -& MORENO ANA BEATRIZ ET AL: "Pathogen-induced production of the antifungal AFP protein from Aspergillus giganteus confers resistance to the blast fungus Magnaporthe grisea in transgenic rice", MOLECULAR PLANT-MICROBE INTERACTIONS, Bd. 18, Nr. 9, September 2005 (2005-09), Seiten 960-972, XP002642924, ISSN: 0894-0282
- GRUENER R ET AL: "THE UPSTREAM REGION OF THE GENE FOR THE PATHOGENESIS-RELATED PROTEIN 1A FROM TOBACCO RESPONDS TO ENVIRONMENTAL AS WELL AS TO DEVELOPMENTAL SIGNALS IN TRANSGENIC PLANTS", EUROPEAN JOURNAL OF BIOCHEMISTRY, BERLIN, DE, Bd. 220, Nr. 1, 1994, Seiten 247-255, XP008023965, ISSN: 0014-2956
- SUBRAMANIAM K ET AL: "ISOLATION OF TWO DIFFERENTIALLY EXPRESSED WHEAT ACC SYNTHASE CDNAS AND THE CHARACTERIZATION OF ONE OF THEIR GENES WITH ROOT-PREDOMINANT EXPRESSION", PLANT MOLECULAR BIOLOGY, SPRINGER, DORDRECHT, NL, Bd. 31, 1996, Seiten 1009-1020, XP000619426, ISSN: 0167-4412
- DATABASE EMBL [Online] EBI 05 August 1996 'T.aestivum PAL gene' Retrieved from EBI, accession no. EM_EST:X99705 Database accession no. X99705

## Beschreibung

Die vorliegende Erfindung betrifft einen Pathogen induzierbaren synthetischen Promotor, der zur Regulation der Transkription einer Nukleinsäure geeignet ist und einen Minimalmalpromotor umfasst. Darüber hinaus betrifft die vorliegende Erfindung eine transgene Pflanzenzelle sowie transgene Pflanzen. Die vorliegende Erfindung betrifft weiterhin ein Verfahren zur Herstellung einer gegenüber Pathogenen resistenten Pflanze.

Zur Erstellung von Pflanzen, die resistent gegenüber Pathogenen wie Pilzen, Viren, Bakterien und Nematoden sind, sind verschiedene Verfahren bekannt. Einige dieser Verfahren nutzen die hypersensitive Reaktion (HR) der Pflanze, wobei es an der direkten Kontaktstelle zwischen Pathogen und Pflanze zur Ausbildung von Nekrosen kommt. In Folge der HR wird in den benachbarten Zellen ein breites Spektrum an Pathogenabwehrmaßnahmen eingeleitet, die das weitere Vordringen des Pathogens in das pflanzliche Gewebe verhindern.

Die HR kann nach Expression von Effektorgenen, wie beispielsweise Avirulenzgenen eines Pathogens und Interaktion mit dem Produkt eines korrespondierenden Resistenzgens (R-Gen) erfolgen. Das R-Gen kann hierbei in der Pflanze vorhanden sein bzw. über gentechnologische Methoden in das jeweilige Pflanzengenom eingefügt werden (Stuiver et al. 1998, Keller et al., 1999, Belbahri et al., 2001). Außerdem kann eine Überexpression oder Autoaktivierung von R-Genen zur Auslösung einer HR führen (Tao et al., 2000, Tang et al., 1999, Bendahmane et al., 2002, Howles et al., 2005). Durch die Überexpression eines R-Gens wird ein Grenzwert überschritten, der zur Auslösung einer Signalkaskade führt, die üblicherweise nur bei Anwesenheit des Pathogens bzw. dessen Avirulenzgenproduktes ausgelöst wird. Durch Auslösung dieser Kaskade kann eine breit wirksame Pathogenresistenz erzielt werden (Oldroyd and Staskawicz, 1998, Tang et al., 1999, Tao et al., 2000, Howles et al., 2005). Als autoaktive R-Gene werden solche R-Gene bezeichnet, die insofern modifiziert wurden, dass zur Auslösung der Signalkaskade wiederum nicht die Präsenz des Pathogens/Avirulenzgenproduktes notwendig ist und gleichzeitig eine verringerte Expressionshöhe im Vergleich zur nicht modifizierten Form ausreicht, um die Auslösung der Signalkaskade zu erreichen.

Stuiver et al. (1998) konnte zeigen, dass die Transformation des avr9-Gens aus dem phytopathogenen Pilz *Cladosporium fulvum* unter der Kontrolle des Pathogen induzierbaren Gst1-Promotors aus der Kartoffel in Tomatenpflanzen, die das korrespondierende Cf9-Gen tragen, eine breit wirksame Pilzresistenz herbeiführte. Eine Resistenz gegenüber dem Oomyceten *Phytophthora parasitica* var *nicotianae* konnte in *Nicotiana tabacum* erzielt werden, nachdem entweder der Elizitor Cryptogein aus *P. cryptogea* oder der bakterielle Elizitor popA aus dem phytopathogenen Bakterium *Ralstonia solanacearum* in *N. tabacum* transformiert wurde. Beide Gene standen unter der Kontrolle des Pathogen induzierbaren Promotors hsr203J aus *N. tabacum* (Keller et al., 1999, Belbahri et al., 2001).

Das System der HR-Auslösung verlangt eine stringente Kontrolle der Expression des Effektorgens am Ort der Infektion. Bei unkontrollierter Expression verursacht die Expression des Effektorgens negative Effekte auf das Pflanzenwachstum und damit auf den Ertrag von Kulturpflanzen (Stuiver and Custers, 2001). Eine kontrollierte Expression kann allerdings durch die Auswahl geeigneter, Pathogen induzierbarer Promotoren erfolgen. Diese sollten unter Nichtbefallsbedingungen keine oder aber nur eine geringe Expression zulassen, bei Befall am Ort der Infektion jedoch eine deutlich höhere Expression veranlassen. Nach Transformation von zwei unterschiedlichen autoaktiven Formen des L6 Rostresistenzgens aus Lein (*Linum usitatissimum*) in Lein unter der Kontrolle des natürlichen, durch Rost induzierbaren *Fis1* Promotors aus Lein konnten zwei Phänotypen festgestellt werden. Zum einen normalwüchsige Pflanzen, die keine verbesserte Resistenz gegenüber Pathogenen zeigten, zum anderen kleinwüchsige Pflanzen mit einer breiten Pathogenresistenz (Howles et al., 2005). Diese Ergebnisse zeigen, dass je nach verwendeter Form des autoaktiven R-Gens es zu einer Promotoraktivität kommt, die bereits über dem Grenzwert der Induktion der Signalkaskade liegen kann, während in den phänotypisch unauffälligen Pflanzen die Induktion des *Fis1*-Promotors nicht ausreicht, um den Grenzwert zu erreichen. Die Spezifität des natürlichen *Fis1-*Promotors reicht somit nicht aus, um eine breit wirksame Pathogenresistenz ohne negative Auswirkungen auf das Pflanzenwachstum zu erzielen.

Natürliche, Pathogen induzierbare Promotoren zeigen häufig eine zu unspezifische Aktivität und werden durch zahlreiche Stimuli aktiviert, so dass deren Verwendung für die Expression der oben genannten Effektorgene nicht sinnvoll ist, da eine HR-Auslösung auch unter nicht Infektionsbedingungen stattfinden kann. Diese Undichtigkeit der Promotoren führt zu einer Beeinträchtigung des Pflanzenwachstums und daher zu einer Reduktion des Ernteertrages bei Kulturpflanzen. Daher wurden synthetische Promotoren entwickelt, die Sequenzmotive (cis-regulatorische Elemente) aus natürlichen, Pathogen induzierbaren Promotoren enthalten, die für die Pathogeninduktion relevant sind. Sequenzmotive für andere Stimuli werden dagegen entfernt. Die cis-regulatorischen Elemente wurden stromaufwärts eines Minimalpromotors kloniert, wodurch ein funktioneller Promotor erzeugt wurde, der eine erhöhte Spezifität im Vergleich zu den natürlichen Promotoren aufweist, aus denen die jeweiligen cis-regulatorischen Elemente isoliert wurden (Rushton et al., 2002). Als Minimalpromotor wurde für dikotyle Pflanzen der Bereich -46 bis +8 des 35S-Gens des *Cauliflower Mosaic Virus* verwendet. Außerdem ist die Verwendung eines Minimalpromotors aus einem natürlichen Promotor, aus dem das jeweilige cis-regulatorische Element kloniert wurde, bekannt (Perl-Treves et al., 2004). Für monokotyle Pflanzen ist die Verwendung des Minimalpromotors aus dem Act1-Gen aus Reis beschrieben (Lü et al., 2000).

Obwohl die beschriebenen, synthetischen Promotoren den natürlichen Promotoren überlegen sind, zeigen diese noch immer Hintergrundaktivität auch unter Nichtbefallsbedingungen. Diese Hintergrundaktivität schwankt zwischen einzelnen Pflanzenarten. So kann zwar in allen bisher untersuchten Pflanzenarten eine Pathogeninduzierbarkeit festgestellt werden, jedoch schwanken die Stärke der Induktion und die absolute Aktivität der Promotoren. Durch eine zu starke Hintergrundaktivität in nicht infiziertem Gewebe kann dann nur noch eine geringe Pathogeninduzierbarkeit als Quotient der Promotoraktivität im infizierten Gewebe dividiert durch die Promotoraktivität im nicht infizierten Gewebe festgestellt werden.

Für die Höhe der Hintergrundaktivität eines synthetischen Promotors wurden bislang nur die verwendeten cis-regulatorischen Elemente verantwortlich gemacht. Diese haben einen sehr großen Einfluss auf die Stärke des Promotors (Rushton et al., 2002). Bisher wenig untersucht ist der Einfluss des Minimalpromotors. Nach Literaturangaben hat der Minimalpromotor nur einen sehr geringen Einfluss auf die Regulation der Promotoraktivität (Singh, 1998). Bhullar et al. (2003) konnten jedoch eine deutliche Reduktion der Promotoraktivität des 35S-Promotors feststellen, wenn der Minimalpromotor (-46 bis +1) durch heterologe pflanzliche Minimalpromotoren ausgetauscht wurde. Diese Unterschiede führen sie auf unterschiedliche Sequenzen der TATA-Boxen zurück, während ihrer Meinung nach die flankierenden Bereiche der TATA-Box des Minimalpromotors für die Promotoraktivität nicht relevant waren.

Aufgabe der vorliegenden Erfindung ist es daher, einen Pathogen induzierbaren synthetischen Promotor mit einer geringen Hintergrundaktivität bereitzustellen.

Erfindungsgemäß erfolgt die Lösung der gestellten Aufgabe durch einen Pathogen induzierbaren synthetischen Promotor mit einem Minimalpromotor, wobei der Minimalpromotor ein Sequenzmotiv twcccmt aufweist, das stromabwärts eines TATA-Bereichs und vor einem auf dem Minimalpromotor gelegenen Transkriptionsstartpunkt angeordnet ist, an dem die Transkription der zu regulierenden Nukleinsäure startet, und wobei das Sequenzmotiv in dem Minimalpromotor zwei- oder mehrfach vorkommt und/oder der Minimalpromoter ein Sequenzmotiv twcccmt aufweist, das stromabwärts eines TATA-Bereichs und vor einem auf dem Minimalpromotor gelegenen Transkriptionsstart angeordnet ist, an dem die Transkription der zu regulierenden Nukleinsäure startet, und wobei der Pathogen induzierbare synthetische Promotor neben dem Minimalpromotor mindestens ein cis-regulatorisches Element mit einer Nukleotidsequenz gemäß einer der SEQ ID NOS: 10-15 aufweist.

Die Symbole der Sequenzmotive haben hierbei folgende Bedeutung:
d = Nukleotid a oder g oder t/u
b = Nukleotid c oder g oder t/u
r = Nukleotid g oder a
m = Nukleotid a oder c
w = Nukleotid a oder t/u
a = Nukleotid a
t = Nukleotid t
c = Nukleotid c

Im Sinne dieser Erfindung ist ein "Minimalpromotor" eine DNA-Sequenz eines Promotors, die für die Promotorfunktion erforderlich ist. An dieser DNA-Sequenz können allgemeine Transkriptionsfaktoren, wie z.B. TFII-D, TFII-A, TFII-B, TFII-E und TFII-F, binden und die Plattform für die Bindung des RNA-Polymerase II/TFII-F Komplexes bilden. Da die Transkription der DNA in die mRNA in diesem Bereich startet, liegt der Transkriptionsstartpunkt (TS) innerhalb des Minimalpromotors und wird als Position +1 festgelegt. Der Minimalpromotor umspannt den TS und kann zum Beispiel von Position -50 bis Position +15 reichen. Um die Position -30 wird häufig die so genannte TATA-Box gefunden, die jedoch nicht bei allen Promotoren vorkommt. Die TATA Box ist ein Bereich einer Abfolge von Thymin- und Adenin-Basen. Die TATA-Box ist die Bindungsstelle für das TATA-Box bindende Protein (TBP).

Als "synthetische Promotoren" werden solche Promotoren bezeichnet, die in der Natur nicht vorkommen, aus mehreren Elementen zusammengesetzt sind und einen Minimalpromotor beinhalten sowie stromaufwärts des Minimalpromotors mindestens ein cis-regulatorisches Element aufweisen, welches als Bindungsstelle für spezielle Transkriptionsfaktoren dient. Synthetische Promotoren werden den gewünschten Anforderungen nach konzipiert und werden durch unterschiedliche Faktoren induziert oder reprimiert.

"Derivate" eines Promotors sind verkürzte oder verlängerte oder abschnittsweise identische Versionen dieses Promotors oder Homologe mit gleichen, modifizierten oder singulären Eigenschaften.

Der Ausdruck "Homologie" bedeutet hierbei eine Homologie von mindestens 70 % auf DNA-Ebene, die gemäß bekannter Verfahren, z.B. der computergestützten Sequenzvergleiche (Altschul, S.F. et al., 1990) bestimmt werden kann.

Der erfindungsgemäße Pathogen induzierbare synthetische Promotor führt nach transienter biolistischer Transformation in Blattgewebe der jeweiligen Pflanzen zu einer reduzierten Grundaktivität im Vergleich zu üblicherweise verwendete Promotoren mit einem Minimalpromotor wie dem 35S-Minimalpromotor bei dikotylen Pflanzen und dem Mais-ubi1-Minimalpromotor bei monokotylen Pflanzen. Darüber hinaus wurde gefunden, dass bei den erfindungsgemäßen Pathogen induzierbaren synthetischen Promotoren auch die Induktionsrate erhöht ist.

Die erfindungsgemäßen Pathogen induzierbaren synthetischen Promotoren können somit für die Erstellung von transgenen Pflanzen verwendet werden, die eine breite Resistenz gegenüber zahlreichen Pathogenen, wie Pilzen, Oomyceten, Bakterien, Viren, Insekten und Nematoden, besitzen.

Das Sequenzmotiv twcccmt liegt in sense Orientierung auf dem codogenen Strang zwischen der TATA-Box und dem Transkriptionsstartpunkt und können auch zwei- oder mehrfach vorkommen. Bevorzugte Sequenzen für Minimalpromotoren sind in den SEQ ID NOS: 8 und 9 angegeben.

cis-regulatorische Elemente zur Herstellung von Pathogen induzierbaren synthetischen Promotoren sind vor allem solche Elemente, die in natürlichen Pathogen induzierbaren Promotoren vorkommen und dort für die Pathogeninduktion verantwortlich sind. Ihre Identifizierung ist bei Rushton et al. (2002) beschrieben.

Bevorzugte cis-regulatorische Elemente zur Herstellung von synthetischen Promotoren unter Verwendung der erfindungsgemäßen Minimalpromotoren sind auch in WO 00/29592 beschrieben. Von den dort genannten cis-regulatorischen Elementen eignet sich vor allem die D-Box (SEQ ID NO: 10), insbesondere in der Kombination 2xS/2xD (SEQ ID NO: 11), sowie das Gst1-Element, vorzugsweise in der Kombination 4xGst1 (SEQ ID NO: 12).

Zu den vorteilhaften cis-Elementkombinationen zählen allgemein Kombinationen der D-Box (SEQ ID NO:10) mit der S-Box bzw. dem Gst1-Element. Besonders vorteilhaft sind neben der oben genannten Kombination 2xS/2xD (SEQ ID NO: 11) die Kombinationen 2xS/4xD (SEQ ID NO: 13); 4xS/2xD (SEQ ID NO: 14) und 2xGst1/2xD (SEQ ID NO: 15). Die Kombination des 2xS/4xD Elementes (SEQ ID NO: 13) mit dem Minimalpromotor gemäß SEQ ID NO: 2 zeigte in transgenen Kartoffeln nach Infektion mit *Phytophthora infestans* einen mittleren Anstieg der Reportergenaktivität um den Faktor 253.000 verglichen zur nicht infizierten Kontrolle.

Wurde das Element 4xS/2xD (SEQ ID NO: 14) vor den Minimalpromotor (SEQ ID NO: 2) kloniert, konnte eine mittlere Steigerung der Reportergenaktivität um den Faktor 2.892 festgestellt werden. Beim Element 2xGst1/2xD (SEQ ID NO: 15) wurde eine mittlere Steigerung um den Faktor 2.967 im Vergleich zur Kontrolle erzielt.

Mit den erfindungsgemäßen Promotoren lassen sich somit transgene Pflanzenzellen herstellen, die zu vollständigen Pflanzen mit verbesserten Abwehreigenschaften gegenüber Pathogen regenerieren können. In Saatgut solcher transgenen Pflanzen sind die erfindungsgemäßen Promotoren dann ebenfalls enthalten. Auf bestimmte Pflanzenarten ist diese Erfindung nicht beschränkt.

Die vorliegende Erfindung betrifft daher auch ein Verfahren zur Herstellung einer gegenüber Pathogenen resistenten Pflanze, bei dem in eine Pflanzenzelle ein zur Erzeugung einer Pathogenabwehr geeignetes Gen gebracht wird, das unter der Kontrolle eines Pathogen induzierbaren synthetischen Promotors steht, und anschließend aus dieser Pflanzenzelle eine Pflanze regeneriert wird, dadurch gekennzeichnet, dass es sich bei dem Pathogen induzierbaren synthetischen Promotor um einen Pathogen induzierbaren synthetischen Promotor handelt, wie er oben beschrieben wurde.

### Beispiele

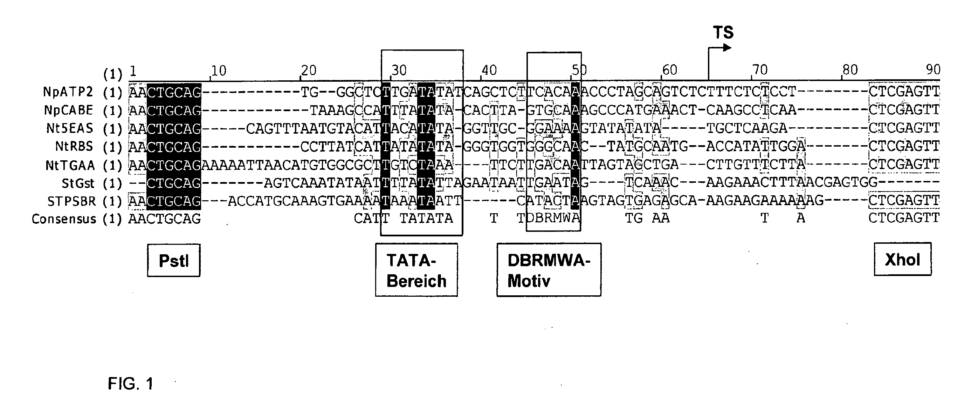

Figur 1 zeigt einen Sequenzvergleich zwischen den für dikotyle Pflanzen bevorzugten Minimalpromotoren (SEQ ID NOS: 1 bis 7) mit den konservierten TATA-Bereichen und den dbrmwa-Motiven sowie den für die Klonierung in das Plasmid pMS23luc+ verwendeten Schnittstellen Pstl und Xhol.

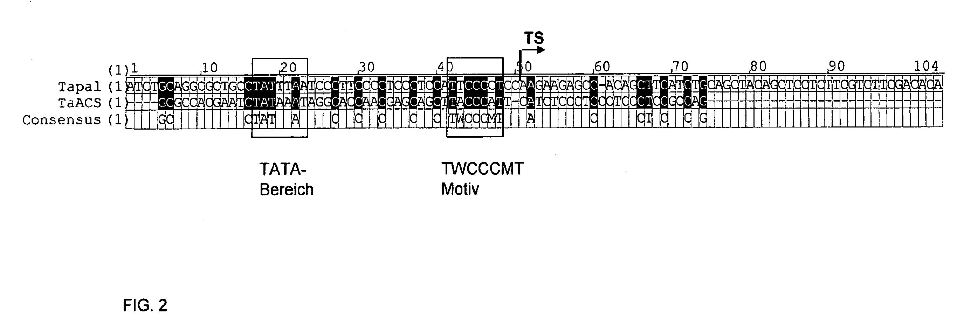

Figur 2 zeigt einen Sequenzvergleich zwischen den für monokotyle Pflanzen bevorzugten Minimalpromotoren (SEQ ID NOS: 8 und 9), die für die transiente Transformation von Weizenblättern verwendet wurden. Neben dem TATA-Bereich ist das Sequenzmotiv twcccmt als konservierter Bereich dargestellt.

Es konnte gezeigt werden, dass die Minimalpromotoren StGst (SEQ ID NO: 6), NtTGAA (SEQ ID NO: 5), StPSBR (SEQ ID NO: 7), NpCABE (SEQ ID NO: 2), NtRBS (SEQ ID NO: 3), NpATP2 (SEQ ID NO: 1) und Nt5EAS (SEQ ID NO: 4) eine deutlich verminderte Aktivität (< 70%) im Vergleich zum 35S-Minimalpromotor aufweisen.

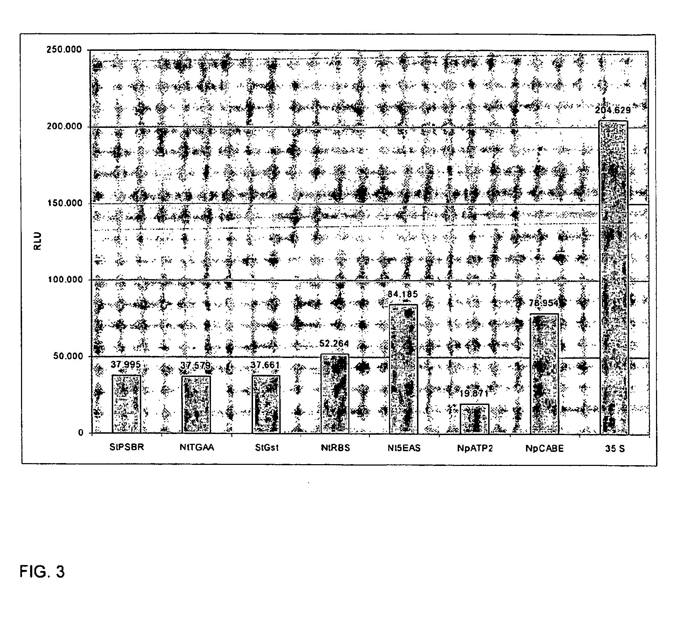

Figur 3 zeigt eine Übersicht über die mittleren Reportergenaktivitäten von nicht infizierten, transgenen Kartoffelpflanzen mit einem synthetischen Promotor bestehend aus dem 4xGst1 Element (SEQ ID NO: 12) und den angegebenen Minimalpromotoren, kloniert vor dem Luciferasegen aus *Photinus pyralis* als Reportergen (RLU = relative Lichteinheiten). Stabile, transgene Linien mit den Minimalpromotoren, die das Sequenzmotiv dbrmwa tragen, zeigten unter Kontrollbedingungen eine deutlich verringerte Expression des Reportergens im Vergleich zum 35S-Minimalpromotor. Die geringste mittlere Aktivität wurde erzielt unter Verwendung des Minimalpromotors des NpATP2 Gens (SEQ ID NO: 1). In diesen Pflanzen konnte nur noch 9,7 % der mittleren Aktivität des 35S-Minimalpromotors gemessen werden. Unter Verwendung der Minimalpromotoren StPSBR (SEQ ID NO: 7), NtTGAA (SEQ ID NO: 5) bzw. StGst (SEQ ID NO: 6) wurden 18 % der Aktivität des 35S-Minimalpromotors gemessen, beim NtRBS-Minimalpromotor (SEQ ID NO: 3) 26 %, beim NpCABE-Minimalpromotor (SEQ ID NO: 2) 39 % und beim Nt5EAS-Minimalpromotor (SEQ ID NO: 4) 41 %.

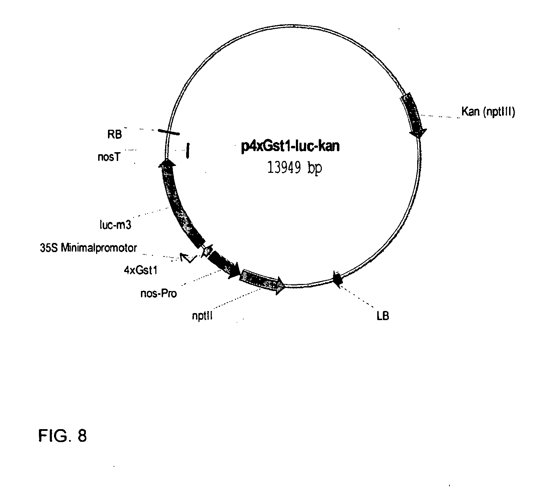

Für den Fachmann ist die Herstellung geeigneter Konstrukte zur Transformation von Pflanzen mit den erfindungsgemäßen Promotoren kein Problem. So lässt sich beispielsweise der Binärvektor p4xGst1-luc-kan (Figur 8) herstellen, der für die stabile Transformation von Kartoffelpflanzen der Sorte "Baltica" verwendet wurde. Dieser Vektor ist ein Derivat des Binärvektors pGPTV (Becker et al., 1992). Der Binärvektor p4xGst1luc-kan trägt das Luciferasegen aus *Photinus pyralis* unter der Kontrolle des synthetischen Promotors 4xGst1:35S Minimalpromotor (Rushton et al., 2002). Als Terminationssequenz enthält das Plasmid den Terminator des Nopalinsynthasegens aus *Agrobacterium tumefaciens.* Die beschriebene Expressionskassette ist lokalisiert auf der T-DNA zusammen mit einer funktionellen Expressionskassette für das Neomycinphosphotransferasegen (nptll) als Selektionsmarker. Die Neomycinphosphotransferase verleiht den transgenen Pflanzen Resistenz gegenüber Kanamycin oder Paromycin. Um den 35S-Minimalpromotor durch die oben beschriebenen Minimalpromotoren auszutauschen, wurde der Binärvektor p4xGst1luc-kan mit Xhol/Sall verdaut, wodurch der 35S-Minimalpromotor entfernt wurde, das Tetramer des Gst1-Elementes jedoch erhalten bleibt. Die Sall-Schnittstelle wurde mit Hilfe des Enzyms Klenow Polymerase und dNTP's aufgefüllt, um ein blunt-end zu erreichen. Die Minimalpromotoren, kloniert in dem Plasmid pMS23luc+, wurden über Pdil/Xhol-Verdau ausgeschnitten und in den Binärvektor ligiert und anschließend in *E. coli* transformiert. Binärvektoren mit der neuen Sequenz wurden in den Agrobakterienstamm GV3101::pMP90 (Koncz and Schell, 1986) transformiert (An, 1987) und unter Verwendung des Antibiotikums Kanamycin (50 mg/l) selektiert. Die transgenen Agrobakterien wurden für die Transformation von Kartoffeln der Sorte "Baltica" eingesetzt (Dietze et al., 1995)

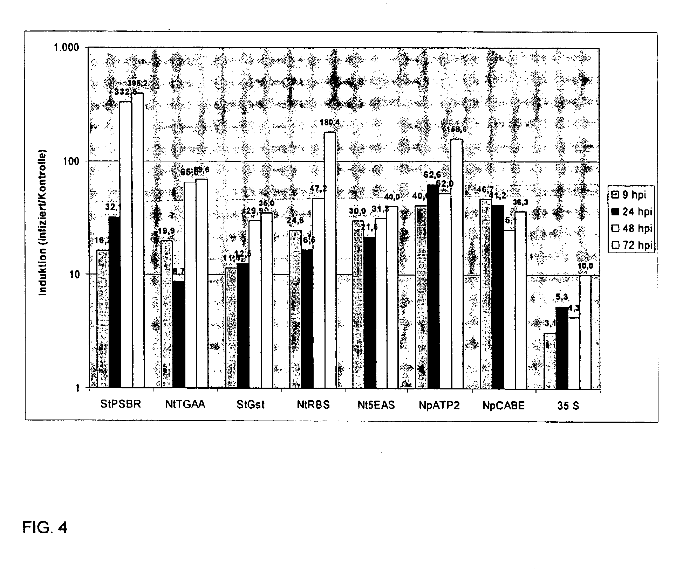

Figur 4 zeigt eine Übersicht über die erzielten Induktionen nach *in vitro* Infektion von stabilen, transgenen Kartoffelpflanzen mit einem synthetischen Promotor bestehend aus dem 4xGst1 Element (SEQ ID NO: 12) und den angegebenen Minimalpromotoren. Die Infektion erfolgte an *in vitro* Pflanzen mit einer Zoosporensuspension von *Phytophthora infestans.*

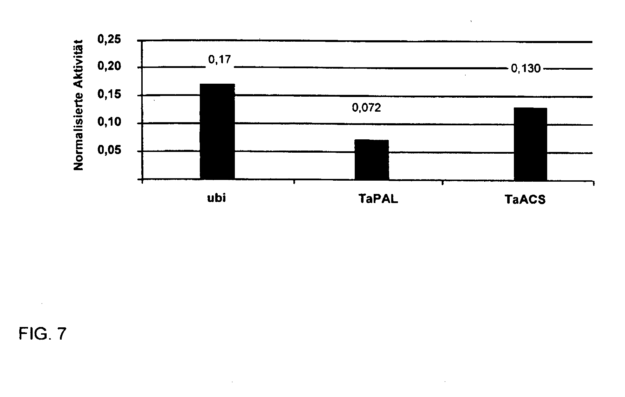

Zu unterschiedlichen Zeitpunkten nach Inokulation wurden Blattproben von *in vitro* Pflanzen abgenommen, das Probengewicht bestimmt und 10 Volumen 1xCCLR Puffer (Promega, Mannheim) zugegeben. Das Material wurde mit Hilfe eines RW20 Homogenisators (IKA Labortechnik, Staufen) im Puffer auf Eis homogenisiert. Durch Zentrifugation bei >10.000 x g für 10 Minuten wurde das Homogenat geklärt und 10 µl des Überstandes wurden mit 50 µl des Substrates LAR (Promega, Mannheim) in einem Luminometerröhrchen versetzt und die Lichtemission als Maß der Aktivität der Luciferase in einem Luminometer (Sirius, Berthold Detection System GmbH, Pforzheim) bestimmt. Zum Vergleich wurden *in vitro* Pflanzen verwendet, die unter gleichen Bedingungen angezogen wurden und anstelle der Zoosporen mit Wasser scheinbehandelt wurden. Der Mittelwert der Quotienten von fünf unabhängigen Linien aus der Luciferaseaktivität in der infizierten zur scheinbehandelten Variante gibt die Induktion des synthetischen Promotors durch die Infektion an. Wie in Abb. 7 zu sehen, konnte unter Verwendung des 35S-Minimalpromotors nur eine maximale Induktion der Luciferaseaktivität um den Faktor zehn 72 h nach Infektion erzielt werden. Alle neuen Minimalpromotoren hingegen zeigten eine deutlich verbesserte Induktion. Die stärkste Induktion nach Infektion mit einem Faktor 395 wurde 72 hpi mit dem StPSBR Minimalpromotor (SEQ ID NO: 7) erzielt. Generell konnte die Induktion durch Verwendung der neuen Minimalpromotoren zum Zeitpunkt 72 hpi um den Faktor 3,5 beim StGst Minimalpromotor (SEQ ID NO: 6) bis zum Faktor 39,5 beim StPSBR Minimalpromotor (SEQ ID NO: 7) im Vergleich zum 35S-Minimalpromotor verbessert werden. Interessanterweise gibt es zwischen den Minimalpromotoren deutliche Unterschiede in der Kinetik der Induktion nach Pathogeninokulation. Während bei Verwendung des 35S-Minmalpromotors 72 hpi die deutlichste Induktion messbar ist, so trifft dies auch bei Verwendung des StPSBR, NtTGAA, StGst, NtRBS sowie NpATP2 Minmalpromotors zu. Beim NpCABE und Nt5EAS Promotor hingegen ist eine starke Aktivierung bereits zum Zeitpunkt 9 hpi feststellbar und die Induktion verbleibt im restlichen Untersuchungszeitraum in etwa auf dem erreichten Niveau.

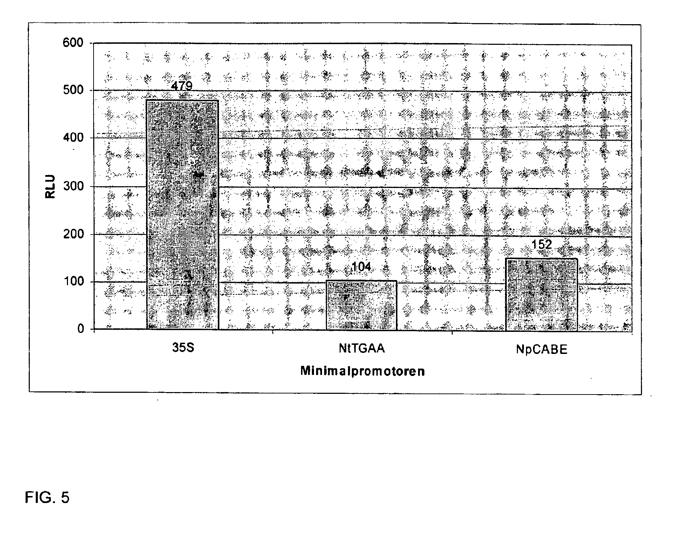

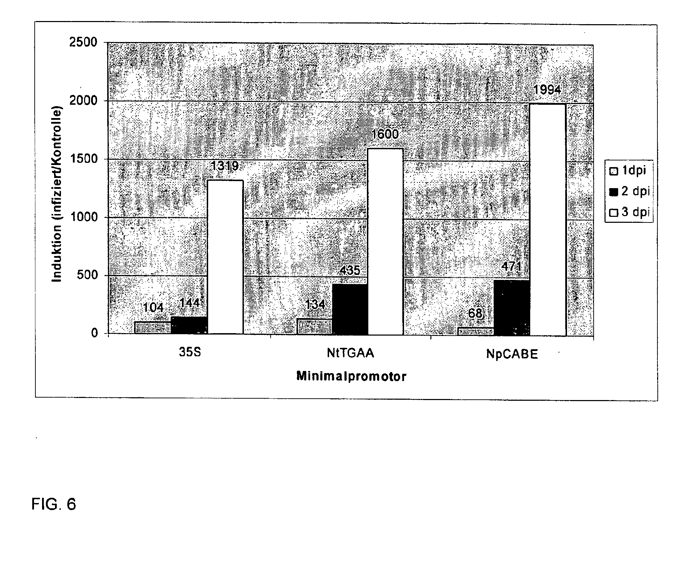

Die Vorzüglichkeit der neuen Minimalpromotoren wurde auch nach Fusion mit der cis-Elementkombination 2xS/2xD gezeigt. Dazu wurden Kartoffelpflanzen stabil mit den Binärvektoren p2xS/2xDluc-kan, p2xS/2xDNpCABEluc-kan und p2xS/2xDNtTGAAluc-kan transformiert. Die Binärvektoren wurden erstellt, indem das 4xGst1-Element aus den oben beschriebenen Binärvektoren mit den neuen Minimalpromotoren und dem 4xGst1-Element über BcuI/Eco147I-Verdau eliminiert und das Element 2xS/2xD (SEQ ID NO:11) als BcuI/Eco32I-Fragment eingefügt wurde. Binärvektoren mit der neuen Sequenz wurden in den Agrobakterienstamm GV3101::pMP90 (Koncz and Schell, 1986) transformiert (An, 1987) und unter Verwendung des Antibiotikums Kanamycin (50 mg/l) selektiert. Die transgenen Agrobakterien wurden für die Transformation von Kartoffeln der Sorte "Baltica" eingesetzt (Dietze et al., 1995). Transgene Sprosse wurden vermehrt und unter *in vitro* Bedingungen mit einer Zoosporensuspension (50.000 Sporen/ ml) von *Phytophthora infestans* inokuliert. Es konnte gezeigt werden, das auch bei Verwendung des cis-regulatorischen Elements 2xS/2xD (SEQ ID NO: 11) eine reduzierte Hintergrundaktivität mit den erfindungsgemäßen Minimalpromotoren im Vergleich zum 35S-Minimalpromotor erzielt werden konnte (Abb. 5). Gleichzeitig konnte eine stärkere Induktion der synthetischen Promotoren nach Inokulation der transgenen Kartoffeln mit *P. infestans* beobachtet werden (Abb. 6). Die Verstärkung der Induktion war zum späten Zeitpunkt (3 Tage nach Infektion = 3 dpi) nicht so deutlich ausgeprägt, wie dies bei Verwendung des 4xGst1-Elements zu beobachten war. 2 Tage nach Infektion konnte jedoch durch die Verwendung der neuen Minimalpromotoren eine deutlich stärkere Induktion nach Pathogenbefall festgestellt werden. Somit hat die Nutzung dieser Minimalpromotoren eine Verbesserung der Kinetik des synthetischen Promotors zur Folge, so dass die Reaktion auf Pathogenbefall früher erfolgt, verglichen mit den synthetischen Promotoren unter Verwendung des 35S-Minimalpromotors.

Figur 7 zeigt den Vergleich der normalisierten Aktivität von Pathogen induzierbaren synthetischen Promotoren bestehend aus dem Element 2xS/2xD (SEQ ID NO: 11) und den Minimalpromotoren ubi1 (Vergleichspromotor), TaPAL (SEQ ID NO: 9) und TaACS (SEQ ID NO: 8) nach biolistischer Transformation in Primärblätter der Weizensorte "Taifun". Wie man erkennen kann, haben die neuen Minimalpromotoren TaPAL und TaACS im Weizen eine reduzierte Grundaktivität im Vergleich zum ubi1-Minimalpromotor. Während mit dem ubi1-Minimalpromotor eine normalisierte Aktivität von 0,17 gemessen wurde, konnte unter Verwendung des TaPAL-Minimalpromotors diese auf 0,072 und durch Verwendung des TaACS-Minimalpromotors auf 0,13 reduziert werden.

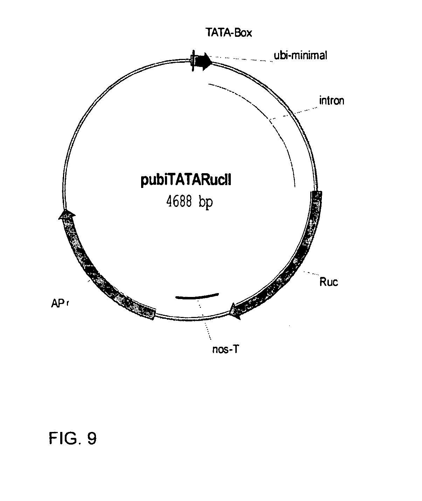

Figur 9 zeigt das Plasmid pubiTATARucll, welches eine cDNA mit dem Luciferasegen aus *Renilla reniformis* enthält, wie sie in dem kommerziell erhältlichen Plasmid pRL-Null vorhanden ist. Die cDNA steht unter der Kontrolle des ubi1-Minimalpromotors. Der ubi1-Minimalpromotor umfasst den Sequenzbereich von -45 bis +76 relativ zum Transkriptionsstartpunkt. Zur Erhöhung der Expressionsstärke ist das erste Intron des ubi1-Gens in seinem natürlichem Kontext in dem Plasmid vor dem Reportergen enthalten. Das Plasmid diente zur Klonierung des cis-regulatorischen Elements 2xS/2xD (SEQ ID NO: 11), um dadurch einen Pathogen induzierbaren synthetischen Promotor zu erstellen. Der ubi1-Minimalpromotor wurde zur Verbesserung der Eigenschaften der synthetischen Promotoren durch die neuen Minimalpromotoren ausgetauscht.

### Referenzen

An, G. (1987). Binary Ti vectors for plant transformation and promoter analysis. Methods Enzymol. 153: 292-305
Altschul, S. F. et al. (1990), Basic Local Alignment search tool, J. Mol. Biol. 215: 403-410
Becker, D. et al. (1992). New plant binary vectors with selectable markers located proximal to the left T-DNA border. Plant Mol Biol. 29: 1195-1197
Belbahri, L. et al. (2001). A local accumulation of the Ralstonia solanacearum PopA protein in transgenic tobacco renders a compatible plant-pathogen interaction incompatible. Plant J. 28: 419-430
Bendahmane, A. et al. (2002). Constitutive gain-of-function mutants in a nucleotide binding site-leucine rich repeat protein encoded at the Rx locus of potato. Plant J. 32: 195-204
Bhullar, S. (2003). Strategies for the development of functionally equivalent promoters with minimum sequence homology for transgene expression in plants: ciselements in a novel DNA context versus domain swapping. Plant Physiol. 132: 988-998
Dietze, J. et al. (1995). Agrobacterium-mediated transformation of potato (Solanum tuberosum). In: Gene Transfer to Plants XXII (Potrykus, I. and Spangenberg, G., eds.). Berlin: Springer Verlag, pp 24-29
Howles, P. et al. (2005). Autoactive alleles of the flax L6 rust resistance gene induce non-race-specific rust resistance associated with the hypersensitive response. Mol. Plant-Microbe Interact. 18: 570-582
Keller, H. et al. (1999). Pathogen-induced elicitin production in transgenic tobacco generates a hypersensitive response and non-specific disease resistance. Plant Cell 11: 223-235
Koncz, C. and Schell, J. (1986). The promoter of TL-DNA gene 5 controls the tissue specific expression of chimeric genes carried by a novel type of Agrobacterium vector. Mol. Gen. Genet., 204: 383-396
Lü, H. et al. (2000). Construction of chimeric inducible promoters by elicitors of rice fungal blast pathogen and their expression in transgenic rice. Chinese Science Bulletin 45: 242-246
Oldroyd, G.E.D. and Staskawicz, B.J. (1998). Genetically engineered broad spectrum disease resistance in tomato. Proc. Natl. Acad. Sci. U. S. A. 95: 10300 - 10350
Maas, C. et al. (1991). The combination of a novel stimulatory element in the first exon of the maize Shrunken-1 gene with the following intron1 enhances reporter gene expression up to 1000-fold. Plant Mol Biol. 16: 199-207
Perl-Treves, R. et al. (2004). Early induction of the Arabidopsis GSTF8 Promoter by specific strains of the fungal pathogen Rhizactonia solani. Mol. Plant-Microbe Interact. 17: 70-80
Rushton, P.J. et al. (2002). Synthetic plant promoters containing defined regulatory elements provide novel insights into pathogen- and wound-induced signalling. Plant Cell 14, 749-762
Singh, K.B. (1998). Transcriptional regulation in plants: the importance of combinatorial control. Plant Physiol. 118: 1111-1120
Stuiver, M. H. and Custers, J.H.H.V. (2001). Engineering disease resistance in plants. Nature 411: 865-868
Stuiver, M.H. et al. (1998). Infection-induced expression of the avirulence gene avr9 in transgenic Cf9 tomato plants confers resistance to fungal pathogen attack. 7th International congress of plant pathology, 9 - 16 August, Edinburgh, Scotland
Tang, X. et al. (1999). Overexpression of Pto activates defense responses and confers broad resistance. Plant Cell 11:15-29
Tao, Y. et al. (2000). Mutational analysis of the Arabidopsis nucleotide binding siteleucine-rich repeat resistance gene RPS2. Plant Cell 12: 2541-2554

### Sequenzprotokoll - freier Text

- SEQ ID NO: 13: synthetisches Enhancerelement bestehend aus Dimer der BoxS und dem Tetramer der BoxD
- SEQ ID NO: 14: cis-Elementkombination 4xS/2xD
- SEQ ID NO: 15: synthetisches Enhancerelement bestehend aus dem Dimer der Gst1-Box und dem Dimer der BoxD

## Patentansprüche

1. Pathogen induzierbarer synthetischer Promotor, der zur Regulation der Transkription einer Nukleinsäure geeignet ist und einen Minimalmalpromotor umfasst, **dadurch gekennzeichnet, dass**
a) der Minimalpromotor ein Sequenzmotiv twcccmt aufweist, das stromabwärts eines TATA-Bereichs und vor einem auf dem Minimalpromotor gelegenen Transkriptionsstartpunkt angeordnet ist, an dem die Transkription der zu regulierenden Nukleinsäure startet, wobei das Sequenzmotiv in dem Minimalpromotor zwei- oder mehrfach vorkommt, und/oder
b) der Minimalpromotor ein Sequenzmotiv twcccmt aufweist, das stromabwärts eines TATA-Bereichs und vor einem auf dem Minimalpromotor gelegenen Transkriptionsstartpunkt angeordnet ist, an dem die Transkription der zu regulierenden Nukleinsäure startet, und wobei der Pathogen induzierbare synthetische Promotor neben dem Minimalpromotor mindestens ein cis-regulatorisches Element mit einer Nukleotidsequenz gemäß einer der SEQ ID NOS: 10-15 aufweist.

2. Pathogen induzierbarer synthetischer Promotor nach Anspruch 1, bei dem der Minimalpromotor eine Nukleotidsequenz gemäß SEQ ID NO: 8 oder SEQ ID NO: 9 aufweist.

3. Minimalpromotor zur Herstellung eines Pathogen induzierbaren synthetischen Promotors, der zur Regulation der Transkription einer Nukleinsäure geeignet ist, **dadurch gekennzeichnet, dass** der Minimalpromotor ein Sequenzmotiv twcccmt aufweist, das stromabwärts eines TATA-Bereichs und vor einem auf dem Minimalpromotor gelegenen Transkriptionsstartpunkt angeordnet ist, an dem die Transkription der zu regulierenden Nukleinsäure startet, wobei das Sequenzmotiv in dem Minimalpromotor zwei- oder mehrfach vorkommt.

4. Minimalpromotor nach Anspruch 3, **dadurch gekennzeichnet, dass** der Minimalpromotor eine Nukleotidsequenz gemäß SEQ ID NO: 8 oder SEQ ID NO: 9 aufweist.

5. Rekombinantes Gen mit einem Pathogen induzierbaren synthetischen Promotor nach Anspruch 1 oder 2 oder einem Minimalpromotor nach Anspruch 3 oder 4.

6. Transgene Pflanzenzelle, bei der ein Pathogen induzierbarer synthetischer Promotor nach Anspruch 1 oder 2 oder ein Minimalpromotor nach Anspruch 3 oder 4 in die DNA der Pflanzenzelle integriert wurde.

7. Transgene Pflanze mit einer Pflanzenzelle nach Anspruch 6.

8. Transgenes Saatgut mit einer Pflanzenzelle nach Anspruch 6.

9. Verfahren zur Herstellung einer gegenüber Pathogenen resistenten Pflanze, bei dem in eine Pflanzenzelle eine zur Erzeugung einer Pathogenabwehr geeignete Nukleinsäure eingebracht wird, die unter der Kontrolle eines Pathogen induzierbaren synthetischen Promotors steht, und anschließend aus dieser Pflanzenzelle eine Pflanze regeneriert wird, **dadurch gekennzeichnet, dass** es sich bei dem Pathogen induzierbaren synthetischen Promotor um einen Pathogen induzierbaren synthetischen Promotor nach Anspruch 1 oder 2 handelt.

## Claims

1. Pathogen inducible synthetic promoter which is suitable for regulating the transcription of a nucleic acid and comprises a minimal promoter, **characterized in that**
a) the minimal promoter has a sequence motif twcccmt which is disposed downstream from a TATA region and in front of a transcription starting point which is located on the minimal promoter and at which transcription of the nucleic acid to be regulated starts, wherein the sequence motif occurs two or more times in the minimal promoter, and/or
b) the minimal promoter has a sequence motif twcccmt which is disposed downstream from a TATA region and in front of a transcription starting point which is located on the minimal promoter and at which transcription of the nucleic acid to be regulated starts, and wherein the pathogen inducible synthetic promoter includes in addition to the minimal promoter at least one cis regulatory element with a nucleotide sequence according to one of SEQ ID NOS: 10-15.

2. Pathogen inducible synthetic promoter according to claim 1, in which the minimal promoter includes the nucleotide sequence according to SEQ ID NOS: 8 or SEQ ID NO: 9.

3. Minimal promoter for the manufacture of a pathogen inducible synthetic promoter which is suitable for regulating the transcription of a nucleic acid, **characterized in that** the minimal promoter has a sequence motif twcccmt which is disposed downstream from a TATA region and in front of a transcription starting point at which transcription of the nucleic acid to be regulated starts, wherein the sequence motif occurs two or more times in the minimal promoter.

4. Minimal promoter according to claim 3, **characterized in that** the minimal promoter includes the nucleotide sequence according to SEQ ID NOS: 8 or SEQ ID NO: 9.

5. Recombinant gene with a pathogen inducible synthetic promoter according to claim 1 or 2 or a minimal promoter according to claim 3 or 4.

6. Transgenic plant cell, in which a pathogen inducible synthetic promoter according to claim 1 or 2 or a minimal promoter according to claim 3 or 4 is integrated into the DNA of the plant cell.

7. Transgenic plant with a plant cell according to claim 6.

8. Transgenic seed with a plant cell according to claim 6.

9. Process for producing a pathogen resistant plant, in which in a plant cell a nucleic acid causing pathogen resistance is introduced, which is under the control of a pathogen inducible synthetic promoter, and subsequently a plant is regenerated from this plant cell, thereby characterized, that the pathogen inducible synthetic promoter is a pathogen inducible synthetic promoter according to claim 1 or 2.

## Revendications

1. Promoteur synthétique inductible par un agent pathogène, qui est apte à la régulation de la transcription d'un acide nucléique et qui comprend un promoteur minimal, **caractérisé en ce que**
a) le promoteur minimal comporte un motif séquentiel twcccmt qui est placé en aval d'une zone TATA sur laquelle démarre la transcription de l'acide nucléique devant être régulé, le motif séquentiel étant présent deux ou plusieurs fois dans le promoteur minimal et/ou
b) le promoteur minimal comporte un motif séquentiel twcccmt qui est placé en aval d'une zone TATA et devant un point d'initiation de la transcription situé sur le promoteur minimal sur laquelle est initiée la transcription de l'acide nucléique qui doit être régulé et le promoteur synthétique inductible par un agent pathogène comportant à côté du promoteur minimal au moins un élément cis-régulateur avec une séquence nucléotide selon l'une des SEQ ID NOS : 10-15.

2. Promoteur synthétique inductible par agent pathogène selon la revendication 1, sur lequel le promoteur minimal comporte une séquence nucléotide selon les SEQ ID NO : 8 ou SEQ ID NO :

3. Promoteur minimal destiné à la production d'un promoteur synthétique inductible par agent pathogène qui est apte à la régulation de la transcription d'un acide nucléique, **caractérisé en ce que** le promoteur minimal comporte un motif séquentiel twcccmt qui est placé en aval d'une zone TATA et devant un point de de départ de la transcription situé sur le promoteur minimal sur laquelle démarre la transcription de l'acide nucléique qui doit être régulé, le motif séquentiel étant présent deux ou plusieurs fois dans le promoteur minimal.

4. Promoteur minimal selon la revendication 3, **caractérisé en ce que** le promoteur minimal comporte une séquence nucléotide selon les SEQ ID NO ; 8 ou SEQ ID NO : 9.

5. Gène recombinant avec un promoteur synthétique inductible par un agent pathogène selon la revendication 1 ou la revendication 2 ou avec un promoteur minimal selon l'une quelconque des revendications 3 ou 4.

6. Cellule végétale transgénique sur laquelle on a intégré un promoteur synthétique Inductible par un agent pathogène selon la revendication 1 ou la revendication 2 ou un promoteur minimal selon la revendication 3 ou la revendication 4 dans l'ADN de la cellule végétale.

7. Plante transgénique avec une cellule végétale selon la revendication 6.

8. Semences transgéniques avec une cellule végétale selon la revendication 6.

9. Procédé destiné à produire une plante résistante aux agents pathogènes, lors duquel on introduit dans une cellule végétale un acide nucléique apte à créer une immunité pathogène qui est sous le contrôle d'un promoteur synthétique inductible par un agent pathogène et ensuite, à partir de ladite cellule végétale, on régénère une plante, **caractérisé en ce que** le promoteur synthétique inductible par un agent pathogène est un promoteur synthétique inductible par un agent pathogène selon la revendication 1 ou la revendication 2.


### SEQUENCE LISTING

<110> KWS SAAT AG
   Schmidt, Klaus
<120> MINIMALPROMOTOR
<130> KWS 0111 PCT/EP/D1
<150> DE102006029129.8
   <151> 2006-06-22
<150> EP07764382.3
   <151> 2007-06-16
<150> PCT/DE2007/001075
   <151> 2007-06-16
<160> 15
<170> PatentIn version 3.5
<210> 1
   <211> 69
   <212> DNA
   <213> Nicotiana plumbaginifolia
<220>
   <221> misc_feature
   <222> (1)..(8)
   <223> PstI Erkennungssequenz
<220>
   <221> TATA_signal
   <222> (19)..(24)
<220>
   <221> misc_feature
   <222> (32)..(37)
   <223> DBRMWA
<220>
   <221> misc_feature
   <222> (32)..(37)
   <223> dbrmwa
<220>
   <221> misc_feature
   <222> (62)..(69)
   <223> XhoI Erkennungssequenz
<400> 1
   aactgcagtg ggctcttgat atatcagctc ttcacaaacc ctagcagtct ctttctctcc      60
   tctcgagtt                                                              69
<210> 2
   <211> 67
   <212> DNA
   <213> Nicotiana plumbaginifolia
<220>
   <221> misc_feature
   <222> (1)..(8)
   <223> PstI Erkennungssequenz
<220>
   <221> TATA_signal
   <222> (19)..(24)
<220>
   <221> misc_feature
   <222> (31)..(36)
   <223> DBRMWA
<220>
   <221> misc_feature
   <222> (31)..(36)
   <223> dbrmwa
<220>
   <221> misc_feature
   <222> (60)..(67)
   <223> XhoI Erkennungssequenz
<400> 2
   aactgcagta aagccattta tatacactta gtgcaaagcc catgaaactc aagcctcaac    60
   tcgagtt                                                              67
<210> 3
   <211> 67
   <212> DNA
   <213> Nicotiana tabacum
<220>
   <221> Misc_feature
   <222> (1)..(8)
   <223> PstI Erkennungssequenz
<220>
   <221> TATA_signal
   <222> (18)..(25)
<220>
   <221> Misc_feature
   <222> (33)..(38)
   <223> DBRMWA
<220>
   <221> Misc_feature
   <222> (33)..(38)
   <223> dbrmwa
<220>
   <221> Misc_feature
   <222> (60)..(67)
   <223> XhoI Erkennungssequenz
<400> 3
   aactgcagcc ttatcattat atatagggtg gtgggcaact atgcaatgac catattggac    60
   tcgagtt                                                              67
<210> 4
   <211> 69
   <212> DNA
   <213> Nicotiana tabacum
<220>
   <221> misc_feature
   <222> (1)..(8)
   <223> PstI Erkennungssequenz
<220>
   <221> TATA_signal
   <222> (24)..(31)
<220>
   <221> misc_feature
   <222> (38)..(43)
   <223> DBRMWA
<220>
   <221> misc_feature
   <222> (38)..(43)
   <223> dbrmwa
<220>
   <221> misc_feature
   <222> (62)..(69)
   <223> XhoI Erkennungssequenz
<400> 4
   aactgcagca gtttaatgta cattacatat aggttgcgga aaagtatata tatgctcaag    60
   actcgagtt                                                            69
<210> 5
   <211> 67
   <212> DNA
   <213> Nicotiana tabacum
<220>
   <221> misc_feature
   <222> (1)..(8)
   <223> PstI Erkennungssequenz
<220>
   <221> TATA_signal
   <222> (19)..(24)
<220>
   <221> misc_feature
   <222> (31)..(36)
   <223> DBRMWA
<220>
   <221> misc_feature
   <222> (31)..(36)
   <223> dbrmwa
<220>
   <221> misc_feature
   <222> (60)..(67)
   <223> XhoI Erkennungssequenz
<400> 5
   aactgcagta aagccattta tatacactta gtgcaaagcc catgaaactc aagcctcaac 60
   tcgagtt 67
<210> 6
   <211> 67
   <212> DNA
   <213> Solanum tuberosum
<220>
   <221> misc_feature
   <222> (1)..(8)
   <223> PstI Erkennungssequenz
<220>
   <221> TATA_signal
   <222> (24)..(30)
<220>
   <221> misc_feature
   <222> (38)..(43)
   <223> DBRMWA
<220>
   <221> misc_feature
   <222> (38)..(43)
   <223> dbrmwa
<400> 6
   aactgcagag tcaaatataa ttttatatta gaataattga atagtcaaac aagaaacttt 60
   aacgagt 67
<210> 7
   <211> 75
   <212> DNA
   <213> Solanum tuberosum
<220>
   <221> misc_feature
   <222> (1)..(8)
   <223> PstI Erkennungssequenz
<220>
   <221> TATA_signal
   <222> (26)..(34)
<220>
   <221> misc_feature
   <222> (36)..(41)
   <223> DBRMWA
<220>
   <221> misc_feature
   <222> (36)..(41)
   <223> dbrmwa
<220>
   <221> misc_feature
   <222> (68)..(75)
   <223> XhoI Restriktionsschnittstelle
<400> 7
   aactgcagac catgcaaagt gaaaataaat aattcatact aagtagtgag agcaaagaag 60
   aaaaaagctc gagtt 75
<210> 8
   <211> 69
   <212> DNA
   <213> Triticum aestivum
<220>
   <221> promoter
   <222> (1)..(69)
   <223> Minimalpromotor
<220>
   <221> TATA_signal
   <222> (13)..(20)
<220>
   <221> misc_feature
   <222> (38)..(44)
   <223> TWCCCMT-Motiv
<220>
   <221> misc_feature
   <222> (38)..(44)
   <223> twcccmt
<400> 8
   gcgccacgaa tctataaata ggcaccaacg agcagcttac ccattcatct ccctccctcc 60
   ctccgccag 69
<210> 9
   <211> 149
   <212> DNA
   <213> Triticum aestivum
<220>
   <221> TATA_signal
   <222> (17)..(23)
<220>
   <221> misc_feature
   <222> (42)..(48)
   <223> TWCCCMT-Motiv
<220>
   <221> misc_feature
   <222> (42)..(48)
   <223> twcccmt
<220>
   <221> 5'UTR
   <222> (51)..(149)
<400> 9
   atctgcaggc gctgcctatt taatcccttc ccctccctcc attcccctcc aagaagagcc 60
   acagcttcat ctgcagctac agctcctctt cgtcttcgac acacaagtat tttttcagga 120
   caaagatcaa tccagataca catacacct 149
<210> 10
   <211> 31
   <212> DNA
   <213> Petroselinum crispum
<220>
   <221> D-box
   <222> (1)..(31)
<400> 10
   tacaattcaa acattgttca aacaaggaac c 31
<210> 11
   <211> 128
   <212> DNA
   <213> Petroselinum crispum
<220>
   <221> enhancer
   <222> (1)..(128)
   <223> synthetisches Enhancerelement bestehend aus Dimer der BoxS und BoxD
<220>
   <221> enhancer
   <222> (1)..(25)
   <223> BoxS
<220>
   <221> enhancer
   <222> (31)..(55)
   <223> BoxS
<220>
   <221> enhancer
   <222> (61)..(91)
   <223> BoxD
<220>
   <221> enhancer
   <222> (98)..(128)
   <223> BoxD
<400> 11
   cagccaccaa acaggaccca gaattctagt cagccaccaa agaggaccca gaattctagt        60
   tacaattcaa acattgttca aacaaggaac ctctagttac aattcaaaca ttgttcaaac       120
   aaggaacc                                                                128
<210> 12
   <211> 118
   <212> DNA
   <213> Solanum tuberosum
<220>
   <221> enhancer
   <222> (1)..(118)
   <223> synthetisches Enhancerelement aus einem Tetramer des Gst1 Elementes
<220>
   <221> enhancer
   <222> (1)..(25)
   <223> BoxGst1
<220>
   <221> enhancer
   <222> (32)..(56)
   <223> BoxGst1
<220>
   <221> enhancer
   <222> (63)..(87)
   <223> BoxGst1
<220>
   <221> enhancer
   <222> (94)..(118)
   <223> BoxGst1
<400> 12
   ttctagccac cagatttgac caaactctag tttctagcca ccagatttga ccaaactcta        60
   gtttctagcc accagatttg accaaactct agtttctagc caccagattt gaccaaac         118
<210> 13
   <211> 202
   <212> DNA
   <213> Artificial Sequence
<220>
   <223> synthetisches Enhancerelement bestehend aus Dimer der BoxS und dem Tetramer der BoxD
<220>
   <221> enhancer
   <222> (1)..(202)
   <223> synthetisches Enhancerelement bestehend aus Dimer der BoxS und dem Tetramer der BoxD
<220>
   <221> enhancer
   <222> (1)..(25)
   <223> Box S
<220>
   <221> enhancer
   <222> (31)..(55)
   <223> Box S
<220>
   <221> enhancer
   <222> (61)..(91)
   <223> Box D
<220>
   <221> enhancer
   <222> (98)..(128)
   <223> Box D
<220>
   <221> enhancer
   <222> (135)..(165)
   <223> Box D
<220>
   <221> enhancer
   <222> (172)..(202)
   <223> Box D
<400> 13
   cagccaccaa agaggaccca gaattctagt cagccaccaa agaggaccca gaattctagt        60
   tacaattcaa acattgttca aacaaggaac ctctagttac aattcaaaca ttgttcaaac       120
   aaggaacctc tagttacaat tcaaacattg ttcaaacaag gaacctctag ttacaattca       180
   aacattgttc aaacaaggaa cc                                                202
<210> 14
   <211> 188
   <212> DNA
   <213> Artificial Sequence
<220>
   <223> cis-Elementkombination 4xS/2xD
<220>
   <221> enhancer
   <222> (1)..(188)
<220>
   <221> enhancer
   <222> (1)..(25)
   <223> Box S
<220>
   <221> enhancer
   <222> (31)..(55)
   <223> Box S
<220>
   <221> enhancer
   <222> (61)..(85)
   <223> Box S
<220>
   <221> enhancer
   <222> (91)..(115)
   <223> Box S
<220>
   <221> enhancer
   <222> (121)..(151)
   <223> Box D
<220>
   <221> enhancer
   <222> (158)..(188)
   <223> Box D
<400> 14
   cagccaccaa agaggaccca gaattctagt cagccaccaa agaggaccca gaattctagt       60
   cagccaccaa agaggaccca gaattctagt cagccaccaa agaggaccca gaattctagt      120
   tacaattcaa acattgttca aacaaggaac ctctagttac aattcaaaca ttgttcaaac      180
   aaggaacc                                                               188
<210> 15
   <211> 130
   <212> DNA
   <213> Artificial Sequence
<220>
   <223> synthetisches Enhancerelement bestehend aus dem Dimer der Gst1-Box und dem Dimer der BoxD
<220>
   <221> enhancer
   <222> (1)..(130)
   <223> synthetisches Enhancerelement bestehend aus dem Dimer der Gst1-Box und dem Dimer der BoxD
<220>
   <221> enhancer
   <222> (1)..(25)
   <223> Box Gst1
<220>
   <221> enhancer
   <222> (32)..(56)
   <223> Box Gst1
<220>
   <221> enhancer
   <222> (63)..(93)
   <223> Box D
<220>
   <221> enhancer
   <222> (100)..(130)
   <223> Box D
<400> 15
   ttctagccac cagatttgac caaactctag tttctagcca ccagatttga ccaaactcta       60
   gttacaattc aaacattgtt caaacaagga acctctagtt acaattcaaa cattgttcaa      120
   acaaggaacc                                                             130